# EUROPÄISCHE PATENTANMELDUNG

(11) **EP 1 136 043 A2**
(43) Veröffentlichungstag der Anmeldung: **26.09.2001**
(21) Anmeldenummer: 01250080.7
(22) Anmeldetag: 10.03.2001
(51) Int. Cl.: A61F 2/06

(54) **Stent**

(30) Priorität: 15.03.2000 DE 10012460
(71) Anmelder: BIOTRONIK Mess- und Therapiegeräte GmbH & Co Ingenieurbüro Berlin, 12359 Berlin (DE)
(72) Erfinder: Müller, Heinz, 91054 Erlangen (DE); Harder, Claus, 91080 Uttenreuth (DE)
(74) Vertreter: Eisenführ, Speiser & Partner

(57) **Zusammenfassung**

Stent, insbesondere Koronarstent, mit wenigstens zwei in Längsrichtung des Stents (1;) benachbart angeordneten, rohrförmigen Abschnitten (8) aus mehreren miteinander verbundenen, im wesentlichen zellenförmigen und eine Orientierung aufweisenden Elementen (10), die in Längsrichtung des Stents (1) über mindestens ein erstes Verbindungsmittel (20) miteinander verbunden sind, wobei die Elemente (10) derart angeordnet und/oder ausgebildet sind, dass die Längsrichtung des Stents liegenden Enden der Elemente (10) eine in Umfangsrichtung des Stents wellenartig umlaufende Randkontur (36, 37, 38) definieren, und wobei die aneinander angrenzenden Randkonturen (36, 37) zweier rohrförmiger Abschnitte (8) im wesentlichen phasengleich umlaufen.

## Beschreibung

Die Erfindung betrifft einen Stent, insbesondere einen Koronarstent, mit wenigstens zwei in Längsrichtung des Stents benachbart angeordneten, rohrförmigen Abschnitten aus mehreren miteinander verbundenen, im wesentlichen zellenförmigen und eine Orientierung aufweisenden Elementen, die in Längsrichtung des Stents über mindestens ein erstes Verbindungsmittel miteinander verbunden sind, wobei die Elemente derart angeordnet und/oder ausgebildet sind, dass die Längsrichtung des Stents liegenden Enden der Elemente eine in Umfangsrichtung des Stents wellenartig umlaufende Randkontur definieren.

Stents sind aus dem Stand der Technik in vielfältiger Weise bekannt. Sie werden unter anderem in Zusammenhang mit der perkutanen transluminalen Angioplastie (PTCA = percutaneous transluminal coronary angioplasty) in der Gefäßchirurgie des Herzens verwendet. Stents können jedoch auch dazu dienen, andere Körperöffnungen aufzuweiten oder aufgeweitet zu halten. Diesen medizinischen Verfahren geht zunächst die Bestimmung des Ortes der Verengung des jeweiligen Gefäßes voraus. Anschließend wird beispielsweise bei der PTCA ein sogenannter Angioplastieballon in die Arterie, welche die Verengung, die so genannte Stenose, aufweist, geschoben, und an den Ort der Stenose gebracht. Anschließend wird der Ballon expandiert, so dass durch die radial nach außen gerichtete Kraft des Ballons die Stenose aufgeweitet und im optimalen Fall der ursprüngliche Durchtrittsquerschnitt der zuvor verengten Arterie wiederhergestellt ist.

Um im Anschluss an die Aufweitung den aufgeweiteten Zustand der Arterie aufrechtzuerhalten, wurden die eingangs genannten Stents entwickelt. Die Stents dienen somit dazu, das erneute Auftreten einer Stenose zu verhindern. Der Erfolg des so genannten Stenting hängt jedoch unter anderem davon ab, wie gleichförmig sich der Stent an die Innenseite der Gefäßwand anlegen kann. Denn umso gleichförmiger die Gefäßwand durch den Stent abgestützt werden kann, um so wahrscheinlicher ist es, dass es im Bereich des eingesetzten Stents nicht erneut zu Gefäßverengungen kommen kann. Somit bewirkt eine gleichmäßige Stentstruktur eine relativ glatte Gefäßinnenoberfläche, an welche sich Blutpartikel nur schwer anlagern können. Darüber hinaus werden auch Wucherungen der Intima in das Gefäßinnere durch den Stent hindurch stärker durch eine gleichmäßige Stentstruktur verhindert.

Stents mit der vorerwähnten geschlossenen Struktur sind aus dem Stand der Technik bekannt. Aus der US 4,655,771 ist beispielsweise ein Stent bekannt, der sogenannte Wallstent, der eine geschlossene Struktur aufweist, die aus zwei gleichmäßig maschenartig gewirkten, in Längsachse des Stents spiralförmig verlaufenden Drähten gebildet ist.

Der Vorteil der geschlossenen Struktur bei der Abdeckung der Gefäßinnenwand wird jedoch durch den Nachteil erkauft, dass diese Stents eine relative longitudinale Steifheit während des Einsetzens des Stents insbesondere in enge Blutgefäße aufweisen. Diese Stents erlauben es daher nicht in optimaler Weise, den Stent beim Einführen in Richtung auf die zu behandelnde Stenose durch möglicherweise sehr stark gekrümmte Gefäßabschnitte der Herzkranzarterien einzuführen. Auch ist es bei geschlossenen Strukturen problematisch, diese aufgrund ihrer longitudinalen Steifheit im Bereich von kurvigen Gefäßabschnitten zu verwenden. Besonders nachteilig ist bei einer geschlossenen Struktur wie dem Wallstent im übrigen, dass eine starke Längskontraktion bei der Dilatation eintritt.

Um die Nachteile mit Stents mit einer geschlossenen Struktur zu vermeiden, wurden Stents entwickelt, die einen sogenannten modularen Aufbau aufweisen. Bei diesen modulartig aufgebauten Stents sind einzelne, mit einer geschlossenen Struktur versehene Abschnitte durch flexible Verbindungen miteinander verbunden. Derartige Stents sind beispielsweise aus dem US 5,104,404 bekannt.

Weiterhin ist ein gattungsgemäßer Stent mit der Bezeichnung Tenax® bekannt (Handbook of Coronary Stents, Martin Dunitz, Ltd., London, 1998, S. 121ff.). Bei diesem verlaufen die benachbarten Randbereiche benachbarter rohrförmiger Abschnitte aus Gründen der Längenkompensation in Umfangsrichtung des Stents um eine halbe Periode zueinander versetzt. Hierdurch wird eine Kompensation der Verkürzung des Stents erzielt, welche beim Expandieren aus der Verkürzung der zellenförmigen Elemente in Längsrichtung des Stents resultiert.

Nachteilig bei den bekannten modularen bzw. segmentierten Stents ist es jedoch, dass sie im expandierten Zustand oftmals relativ große, gerade die segmentierten Stents auch unterschiedlich große Lücken in dem abgedeckten Bereich der Gefäßwand aufweisen. Durch diese im expandierten Zustand des Stents mehr oder weniger ungleichmäßige Abdeckung der Gefäßinnenwand durch die Stege des Stents, und vor allem auch durch die relativ großen freibleibenden Flächen zwischen den Stegen des Stents, kann das Gewebe der Gefäßinnenwand nach der Implantation des Stents in das Gefäß hinein prolabieren, was zu einer erneuten Verengung des Gefäßes, einer sogenannten Restenose, führen kann.

Aufgabe der vorliegenden Erfindung ist es daher, einen Stent der eingangs genannten Art derart weiterzuentwickeln, dass die vorgenannten Nachteile vermieden werden und eine Abdeckung der Gefäßinnenwand erreicht wird, deren freibleibende Flächen gegenüber dem Stand der Technik verkleinert sind.

Diese Aufgabe wird ausgehend von einem Stent gemäß dem Oberbegriff des Anspruchs 1 durch die im kennzeichnenden Teil des Anspruchs eines angegebenen Merkmale gelöst.

Der vorliegenden Erfindung liegt die technische Lehre zu Grunde, dass man eine besonders gute Abdeckung der Gefäßinnenwand des betroffenen Gefäßes erzielt, wenn die Elemente derart angeordnet und zusätzlich oder alternativ derart ausgebildet sind, dass die aneinander angrenzenden Randkonturen zweier rohrförmiger Abschnitte im wesentlichen phasengleich umlaufen. Durch die in Phase umlaufenden Randkonturen ist es möglich, die beiden aneinander grenzenden rohrförmigen Abschnitte so nahe aneinander anzuordnen, dass zum einen schon im nicht expandierten, vor allem aber im expandierten Zustand des Stents kleinere Lücken in der Stentstruktur entstehen. Zum anderen entstehen hierbei weiterhin in vorteilhafter Weise gleichmäßigere Lücken in der Stentstruktur. Mit anderen Worten ergibt sich sich aufgrund der Erfindung eine gegenüber dem Stand der Technik über die gesamte Mantelfläche des expandierten Stents gleichmäßigere Abdeckung der Gefäßinnenwand, was sich günstig auf die Restenoserate auswirkt.

Die erfindungsgemäßen Vorteile kommen dann besonders zum Tragen, wenn der Stent in einem gekrümmten Gefäß expandiert wird, da in einem solchen Fall die Abschnitte an der Krümmungsaußenseite des in dem gekrümmten Gefäßexpandierten Stents zu einem Auseinanderklaffen neigen. Der Einfluss dieses Auseinanderklaffens wird durch die erfindungsgemäße Gestaltung vorteilhaft unterdrückt.

Es hat sich gezeigt, dass sich auch mit der erfindungsgemäßen Gestaltung durch entsprechende Anordnung der ersten Verbindungsmittel sowie entsprechende Gestaltung der Verbindungen zwischen den Elementen eines rohrförmigen Abschnittes eine ausreichende Verkürzungskompensation beim Expandieren erzielen lässt.

Die Entfernung zwischen den rohrförmigen Abschnitten in Längsrichtung des Stents kann je nach erforderlicher Flexibilität bzw. je nach den für den Einsatzzweck geforderten erzielbaren minimalen Krümmungsradien des Stents gewählt sein. Je geringer dieser Abstand ist, desto eher berühren sich beim Krümmen des Stents allerdings die Elemente benachbarter rohrförmiger Abschnitte, wodurch dann eine weitere Krümmung behindert ist bzw. nur unter Übereinanderschieben der Elemente möglich ist.

Bei bevorzugten Varianten des erfindungsgemäßen Stents sind die die Elemente derart angeordnet und zusätzlich oder alternativ derart ausgebildet, dass die Randkonturen der beiden benachbarten rohrförmigen Abschnitte nach Art einer Verzahnung ineinander greifen. Hierdurch wird eine besonders gute, d. h. gleichmäßige Abdeckung der Gefäßinnenwand mit kleinen Lücken relativ gleichmäßiger Größe erzielt.

Hierbei soll der Begriff "nach Art einer Verzahnung" so verstanden werden, dass sich die Elemente benachbarter rohrförmiger Abschnitte hierbei nicht notwendigerweise berühren. Vielmehr kommen bei dieser Ausführungsform die einzelnen Elemente des einen rohrförmigen Abschnittes mit anderen Worten bevorzugt so nahe an die Elemente des benachbarten rohrförmigen Abschnittes heran, dass sich die benachbarten Elemente in Längsrichtung des Stents teilweise überlappen oder nahezu überlappen. Ein derartiges Überlappen oder teilweises Überlappen benachbarter Elemente benachbarter Abschnitte ist vor allen Dingen dann vorteilhaft, wenn es beim Dilatieren des Stents in Längsrichtung zu einer Verschiebung der einzelnen Elemente kommt.

Die beiden Randkonturen eines rohrförmigen Abschnitts verlaufen bei bevorzugten Varianten des erfindungsgemäßen Stents zueinander im wesentlichen in Phase. Bei anderen vorteilhaften Varianten verlaufen sie zueinander im wesentlichen um eine halbe Periode versetzt. Hierbei versteht es sich, dass innerhalb des Stents gegebenenfalls auch rohrförmige Abschnitte dieser beiden Varianten miteinander kombiniert werden können.

Die erfindungsgemäße Gestaltung der Randkonturen der rohrförmigen Abschnitte lässt sich auf verschiedene Weise realisieren. So können in dem jeweiligen rohrförmigen Abschnitt beispielsweise Elemente mit in Längsrichtung des Stents im wesentlichen gleichen Abmessungen abwechselnd in Längsrichtung des Stents zueinander versetzt angeordnet sein, sodass sich eine in Umfangsrichtung des Stents wellenförmige Anordnung dieser Elemente ergibt. Bei diesen Varianten ist mit anderen Worten die Länge der Elemente der rohrförmigen Abschnitte in Längsrichtung des Stents kleiner ist als die in Längsrichtung des Stents gemessene maximale Breite des entsprechenden rohrförmigen Abschnittes. Hiermit ist dann eine rohrförmige Abschnitte realisiert, dessen Randkonturen miteinander in Phase verlaufen.

Randkonturen, welche zueinander im wesentlichen um eine halbe Periode versetzt verlaufen, lassen sich beispielsweise dadurch erzielen, dass in Umfangsrichtung des Stents wechselweise Elemente angeordnet sind, die in Längsrichtung des Stents unterschiedliche Abmessungen aufweisen. Hierbei lassen sich die Amplituden der Randkonturen einfach dadurch einstellen, dass die Positionierung der einzelnen Elemente zueinander bezüglich der Längsrichtung entsprechend gewählt wird. Gleiche Amplituden lassen sich beispielsweise dadurch einstellen, dass die Mittelpunkte der einzelnen Elemente in Umfangsrichtung des Stents miteinander fluchten.

Bei bevorzugten Varianten des erfindungsgemäßen Stents verbindet das jeweiligen erste Verbindungsmittel Elemente gleicher Orientierung miteinander. Dies ist insbesondere dann von Vorteil, wenn die einzelnen Elemente nicht symmetrisch ausgebildet sind, sondern eine in Längsrichtung des Stents asymmetrische Geometrie aufweisen. Der Vorteil liegt hierbei in dem prinzipiell geringeren Ausmaß der durch die Expansion des Stents bedingten Verkürzung des Stents. Dieser Effekt ist dadurch bedingt, dass die ersten Verbindungsmittel jeweils unterschiedliche Enden der asymmetrischen, eine Orientierung aufweisenden Elemente miteinander verbinden, während im Stand der Technik bei asymmetrischen, eine Orientierung aufweisenden Elementen stets die gleichen Enden dieser Elemente miteinander verbunden werden.

Dieser Vorteil kommt insbesondere bei schlüssellochförmig ausgebildeten Elementen zum Tragen, da in diesem Fall bei der Erfindung die kleinen Bögen des schlüssellochförmigen Elementes mit den großen Bögen des benachbarten schlüsselförmigen Elementes verbunden werden. In einem solchen Fall erfahren die kleinen Bögen bei der Expansion eine größere Verschiebung entlang der Längsachse des Stents erfahren als die größeren Bögen. Während beim Stand der Technik jeweils die kleinen Bögen miteinander verbunden sind und demgemäß eine relativ hohe, in einer starken Verkürzung des Stents resultierende Verschiebung in Längsrichtung des Stents erfahren, ist dieser Effekt bei der erfindungsgemäßen Gestaltung reduziert. Somit ergibt sich bei der erfindungsgemäßen Lösung eine geringere expansionsbedingte Verkürzung des Stents. Auf diese Weise kann die durch die Expansion des Stents bedingte Verkürzung des Stents auf ein Minimum reduziert werden.

Durch gleichzeitiges bevorzugtes wechselweises Orientieren der einzelnen Elemente jedes Abschnittes in einer um 180° versetzten Weise wird die erwähnte weelenartige Anordnung der einzelnen Elemente jedes rohrförmigen Abschnittes in Umfangsrichtung gesehen erreicht. Bei dieser Ausführungsform können die einzelnen Elemente des Abschnittes, wie erwähnt, so nahe an die Elemente des benachbarten Abschnittes herankommen, dass sich die benachbarten Elemente in Längsrichtung des Stents teilweise überlappen oder nahezu überlappen. Ein derartiges Überlappen oder teilweises Überlappen benachbarter Elemente benachbarter Abschnitte ist vor allen Dingen dann vorteilhaft, wenn es beim Dilatieren des Stents in Längsrichtung zu einer Verschiebung der einzelnen Elemente kommt.

Das das erste Verbindungsmittel kann beliebig ausgebildet sein. Bevorzugt ist es wie auch die Elemente selbst stegartig ausgebildet. Ebenso kann es eine beliebige Anordnung bezüglich der Längsrichtung des Stents aufweisen. Bevorzugt verläuft das erste Verbindungsmittel im wesentlichen parallel zur Längsachse des Stents.

Bevorzugte, weil äußerst flexible Varianten des erfindungsgemäßen Stents zeichnen sich dadurch aus, dass möglichst wenige, höchstens zwei, bevorzugt nur ein erstes Verbindungsmittel zum Verbinden benachbarter rohrförmiger Abschnitte vorgesehen sind bzw. ist. Bei dieser Ausführungsform, die bevorzugt mit rohrförmigen Abschnitten mit acht Elementen eingesetzt wird, wird der Stent aufgrund der geringen Anzahl von Verbindungsmitteln vorteilhaft flexibel gehalten.

Bei vorteilhaften Ausführungsformen des erfindungsgemäßen Stents sind die ersten Verbindungsmittel über die Länge des Stents von Abschnitt zu Abschnitt jeweils miteinander fluchtend ausgebildet. Auf diese Weise ergibt sich eine besonders gleichmäßige Abdeckung der Gefäßinnenoberfläche. Dabei ist weiter bevorzugt jeder rohrförmige Abschnitt identisch ausgebildet, so dass die Gleichmäßigkeit des erfindungsgemäßen Stents weiter erhöht wird.

Wird jedoch eine optimale Flexibilität des Stents gewünscht, so werden die ersten Verbindungsmittel in einer alternativen Ausführungsform versetzt zueinander angeordnet. Hierbei sind bevorzugt mehr als zwei rohrförmige Abschnitte vorgesehen und die ersten Verbindungsmittel sind über die Länge des Stents von Abschnitt zu Abschnitt in Umfangsrichtung des Stents versetzt angeordnet. Eine hinsichtlich der Flexibilität des Stents besonders günstige Konfiguration ergibt sich, wenn die erste Verbindungsmittel hierbei um wenigstens eine halbe Periode der Randkontur versetzt angeordnet sind.

Bei einer weiter bevorzugten Ausführungsform der Erfindung sind zweite Verbindungsmittel vorgesehen, die die Elemente jedes Abschnittes in Umfangsrichtung der Stents miteinander verbinden. Diese zweiten Verbindungsmittel sind weiter bevorzugt derart ausgebildet, dass sie mit ihrer Längsachse einen Winkel zur Umfangsrichtung des jeweiligen Abschnittes bilden. Dabei können die Verbindungsmittel gleichzeitig auch S-förmig ausgebildet sein. Insbesondere die Maßnahme der winkelmäßigen Anordnung der Verbindungsmittel sorgt für eine Längsverschiebung der einzelnen Elemente entlang der Längsachse des Stents beim Dilatieren des Stents.

So zeichnen sich weitere bevorzugte Varianten des erfindungsgemäßen Stents dadurch aus, dass die Elemente wenigstens eines rohrförmigen Abschnittes in Umfangsrichtung des Stents über zur Umfangsrichtung geneigt angeordnete, bevorzugt S-förmig verlaufende, zweite Verbindungsmittel verbunden sind, wobei in dieselbe Umfangsrichtung weisende zweite Verbindungsmittel in Längsrichtung des Stents aneinandergrenzender Elemente in entgegengesetzter Weise zur Umfangsrichtung geneigt angeordnet sind. Mit dieser Gestaltung ist es in einfacher Weise möglich, die sich beim Expandieren ergebende Längsverschiebung der einzelnen Elemente, die aus der Ausrichtung der zweiten Verbindungsmittel in Umfangsrichtung resultiert, durch entsprechende versetzte Anordnung der ersten Verbindungsmittel über den gesamten Stent aufzuaddieren und so eine besonders gute Verkürzungskompensation erzielen. Auf diese Weise wird die durch die Expansion des Stents bedingte Verkürzung des Stents auf ein Minimum reduziert.

Die Vorteile der erfindungsgemäßen Lösung liegen insbesondere darin, dass sich auf der Mantelfläche des Stents im expandierten Zustand zwischen benachbarten rohrförmigen Abschnitten nur relativ kleine freie Flächen ergeben, durch die das Gewebe der Gefäßwand hinein in das Gefäß prolabieren könnte. Diese relativ kleinen freien Flächen ergeben sich beibevorzugten Varianten dadurch, dass Elemente identischer Orientierung miteinander verbunden sind. Handelt es sich bei diesen Elementen beispielsweise um aus Stegen schlüssellochförmig gebildete Zellen, so wird aufgrund der Erfindung das breitere Ende des Elementes mit dem schmaleren Ende des zellenförmigen Elementes verbunden, was zu der erfindungsgemäßen und vorteilhaften Verkleinerung derfreibleibenden Fläche im expandierten Zustand des Stents führt.

Besonders vorteilhaft ist es bei der vorliegenden Erfindung, dass nicht nur die Größe der freien Flächen verringert wird, sondern dass insbesondere die Größe von freien Flächen einer bestimmten Form verringert wird. Dabei handelt es sich um solche Flächen innerhalb der Mantelfläche des erfindungsgemäßen Stents, deren geschlossene Umrandung bei einem Umlauf Krümmungen lediglich in eine Richtung aufweisen. Insbesondere solche Flächen lassen sich dank der Erfindung in ihrer Größe erheblich gegenüber dem Stand der Technik reduzieren. Und gerade solche Flächen haben sich im Stand der Technik als Stellen erwiesen, in denen das Gewebe leichter in das Gefäßinnere prolabieren kann.

Weiterhin ist es durch die einfachen, jedoch wirkungsvollen Maßnahmen der Erfindung möglich, die aus dem Stand der Technik bekannten Vorteile segmentierter Stents, beispielsweise des Stents "Tenax® " der Anmelderin, zu erhalten. Insbesondere bleiben die guten mechanischen Eigenschaften (recoil, Kollapsdruck, longitudinale Flexibilität, niedriges Crimp-Profil) erhalten, da sich gerade im Vergleich zu dem bekannten "Tenax® "-Stent die Struktur der rohrförmigen Abschnitte selbst sowie die grundsätzliche Art der Verbindung benachbarter Abschnitte nicht ändert.

Die vorliegende Erfindung betrifft weiterhin eine Dilatationskatheter mit einem erfindungsgemäßen Stent.

Weitere vorteilhafte Ausführungsformen der Erfindung ergeben sich aus den Unteransprüchen oder anhand der nachstehenden Beschreibung bevorzugte Ausführungsbeispiele, welche auf die beigefügten Zeichnungen Bezug nimmt. Es zeigen:
- Figur 1: eine Abwicklung eines Teils der Mantelfläche eines erfindungsgemäßen Stents;
- Figur 2: schematisch die Abwicklung aus Figur 1 in einem nicht expandierten (schwache Linien) und einem expandierten (kräftige Linien) Zustand;
- Figur 3: einen Stent aus dem Stand der Technik anhand der Abwicklung seiner Mantelfläche;
- Figur 4: eine schematische Darstellung des Stents der Figur 3 in nicht-expandiertem (gepunktete Linien) und expandiertem (kräftige Linien) Zustand;
- Figur 5: einen schematischen Querschnitt durch einen Stent gemäß Figur 1 im Bereich der Linie römisch V-V aus Figur 1;
- Figur 6: einen schematischen Querschnitt durch einen erfindungsgemäßen Stent, bei dem benachbarte Abschnitte um 45° gegeneinander verdreht sind;
- Figur 7: eine Abwicklung eines Teils der Mantelfläche einer weiteren Variante des erfindungsgemäßen Stents;
- Figur 8: eine Abwicklung eines Teils der Mantelfläche einer anderen Variante des erfindungsgemäßen Stents;
- Figur 9: eine Abwicklung eines Teils der Mantelfläche einer weiteren Variante des erfindungsgemäßen Stents;
- Figur 10: eine schematische Abwicklung eines Teils der Mantelfläche einer weiteren Variante des erfindungsgemäßen Stents;
- Figuren 11a bis 11d: verschiedene Formen von Elementen für die rohrförmigen Abschnitte eines erfindungsgemäßen Stents.

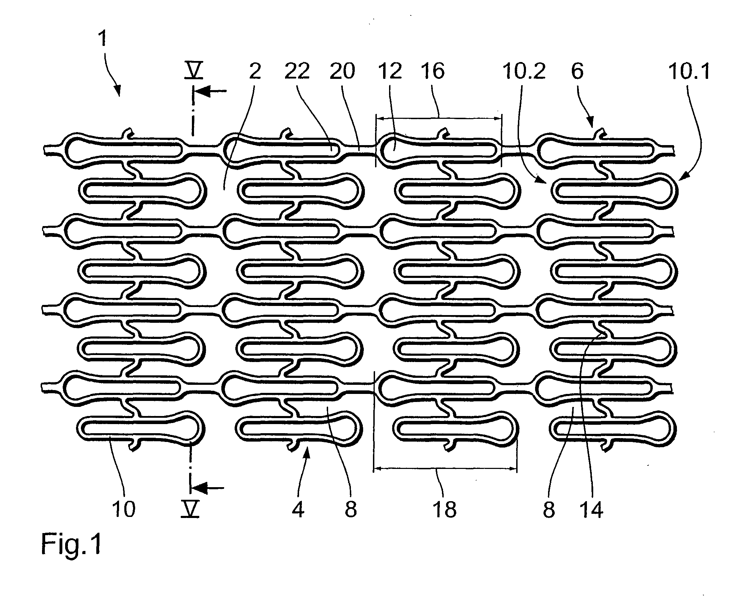

Die Figur 1 zeigt einen erfindungsgemäßen Stent 1. Der Stent 1 ist in der Figur 1 in Form eines Teils der Abwicklung seiner Mantelfläche 2 dargestellt, Im funktionsfähigen Zustand des Stents 1 ist die Mantelfläche 2 mit ihrer in der Figur 1 unten dargestellten Seite 4 mit der in der Figur 1 oben dargestellten Seite 6 verbunden, so dass sich der funktionsfähige rohrförmige Stent 1 ergibt.

Die Mantelfläche 2 setzt sich aus vier rohrförmigen Abschnitten 8 zusammen, die in der Figur 1 ebenfalls abgewickelt dargestellt sind. Jeder rohrförmige Abschnitt 8 weist in der Figur 1 acht zellenförmige, asymmetrisch in der Form eines Schlüsselloches ausgebildete, eine Orientierung aufweisende Elemente 10 auf. Die zellenförmigen Elemente 10 sind in jedem rohrförmigen Abschnitt 8 jeweils mit ihren Längsachsen parallel zur Längsachse des Stents 1 ausgerichtet, in Umfangsrichtung des Stents 1 benachbart zueinander angeordnet. Jedes Element 10 weist eine identische Grundform auf. Die Elemente 10 sind jedoch so angeordnet, dass die schlüssellochartige Grundform der Elemente 10 bei benachbarten Elementen 10 jeweils eine entgegengesetzte Orientierung aufweist, d.h. die ausgebauchten Enden 12 der Elemente 10 sind bei benachbarten Elementen 10 jeweils um 180° zueinander verdreht.

Die in Umfangsrichtung des Stents 1 benachbarten Elemente 10 werden über S-förmige, als zweite Verbindungsmittel dienende zweite Verbindungsstege 14 miteinander in Umfangsrichtung des Stents 1 verbunden. Die S-förmigen zweiten Verbindungsstege 14 sorgen dafür, dass benachbarte Elemente 10 jeweils in Längsrichtung des Stents 1 ein wenig relativ zueinander verschoben sind. Die Länge 16 der Elemente 10 jedes rohrförmigen Abschnittes 8 ist daher jeweils geringer als die Breite 18 jedes röhrförmigen Abschnittes 8. Mithin definieren die in Längsrichtung des Stents 1 liegenden Enden 10.1 bzw. 10.2 der Elemente 10 also für jeden rohrförmigen Abschnitt 8 zwei in Umfangsrichtung des Stents 1 wellenartig, miteinander in Phase umlaufende Randkonturen. Die rohrförmigen Abschnitte 8 sind dabei weiterhin so angeordnet, dass die aneinander angrenzenden Randkonturen benachbarter rohrförmiger Abschnitte 8 phasengleich umlaufen.

Die rohrförmigen Abschnitte 8 sind über als erste Verbindungsmittel dienende erste Verbindungsstege 20 miteinander verbunden. Die ersten Verbindungsstege 20 verlaufen im wesentlichen parallel zur Längsachse des Stents 1. Es versteht sich jedoch, dass bei anderen Varianten der Erfindung die ersten Verbindungsstege auch in anderer Weise ausgebildet und/oder angeordnet sein können. Insbesondere können die ersten Verbindungsstege auch in bekannter Weise einen Verlauf aufweisen, der zur Erhöhung der Flexibilität des Stents beiträgt. So kann der betreffende erste Verbindungssteg beispielsweise ebenfalls S-förmig verlaufen.

Die ersten Verbindungsstege 20 verbinden jeweils benachbarte Elemente 10 der Abschnitte 8 miteinander, indem sie das ausgebauchte Ende 12 eines Elementes 10 mit dem schmalen Ende 22 eines Elementes 10 verbinden. Gemäß Figur 1 ist jedes zweite Element 10 eines rohrförmigen Abschnittes 8 mit dem jeweils in Längsrichtung des Stents 1 benachbarten Element 10 des benachbarten rohrförmigen Abschnittes 8 verbunden. Alle Elemente 10, die über einen ersten Verbindungssteg 20 mit einem benachbarten Element 10 verbunden sind, sind auf der jeweils anderen Seite auch immer mit einem weiteren Element 10 des auf dieser Seite benachbarten Abschnittes 8 verbunden, so dass alle ersten Verbindungsstege 20 in Längsrichtung gesehen miteinander fluchten. Davon ausgenommen sind die in der Figur 1 nicht dargestellten, an den Enden des Stents 1 angeordneten Elemente 10, die zum Rand hin keine weiteren ersten Verbindungsstege 20 aufweisen.

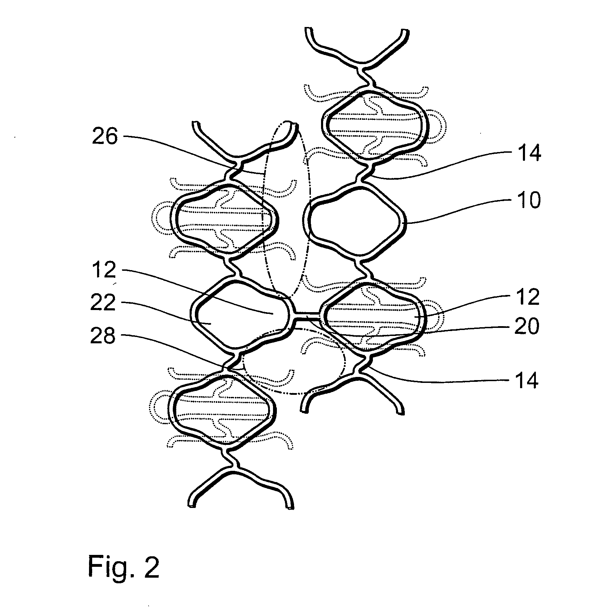

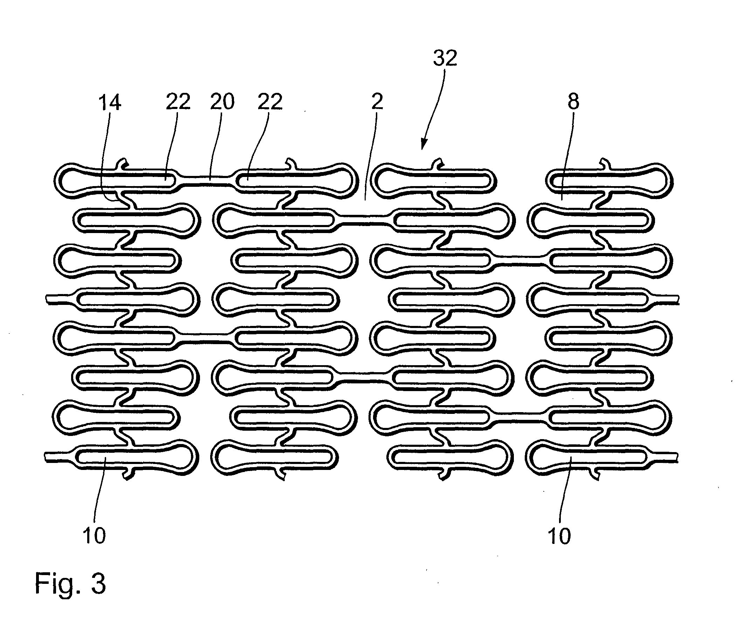

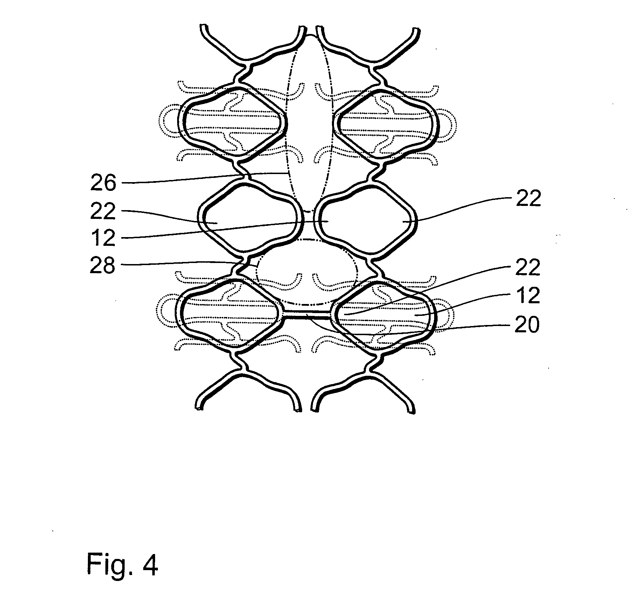

Figur 2 ist eine schematische Darstellung des Stents 1 der Figur 1 in einem nicht expandierten Zustand (gepunktete Linien) und einem expandierten Zustand (kräftige Linien). Teile, die Teilen der Figur 1 entsprechen, sind in der Figur 2 mit gleichen Bezugszeichen bezeichnet. Darüber hinaus sind in der Figur 2 elliptische Flächen 26 und 28 eingezeichnet, die den freien Flächen 26 und 28 eines expandierten, in den Figuren 3 und 4 dargestellten Stents 32 aus dem Stand der Technik entsprechen. Die Figur 2 zeigt deutlich, dass die in der Figur 4 vorhandenen freien Flächen 26 und 28 in expandiertem Zustand eines erfindungsgemäßen Stents 1 nicht mehr in der gleichen Größe vorhanden sind, so dass sich eine Verbesserung der Abdeckung der Gefäßinnenseite ergibt.

Figur 3 zeigt einen Stent 32 aus dem Stand der Technik. Auch hier sind - um die Vergleichbarkeit der Erfindung mit dem Stand der Technik zu erleichtern - Teile, die denen des Stents 1 der Figur 1 im wesentlichen entsprechen, mit gleichen Bezugszeichen bezeichnet. Aus der Figur 3 ergibt sich, dass im Stand der Technik jeweils Elemente 10 entgegengesetzter Orientierung mit Hilfe der Verbindungsmittel 20 verbunden wurden. Auf diese Weise fand die Verbindung mittels des Verbindungssteges 20 jeweils zwischen den schmalen Enden 22 der Elemente 10 statt. Dies hatte im Stand der Technik wiederum die in Figur 4 dargestellten freien Flächen 26 und 28 zur Folge.

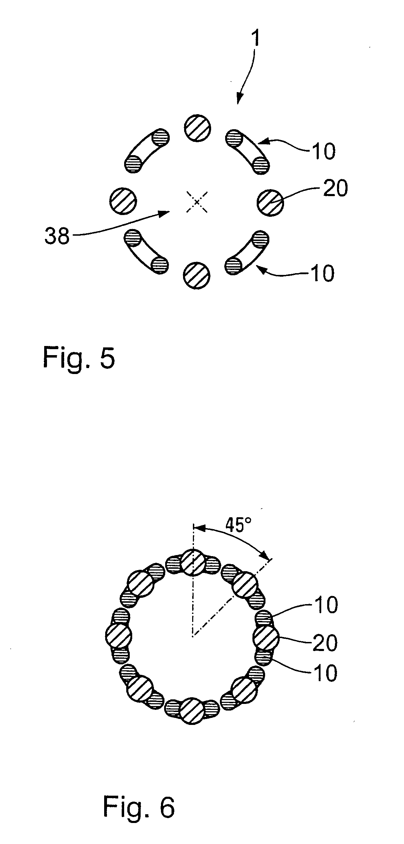

Figur 5 zeigt einen stark schematisierten Querschnitt durch den nicht abgewickelten Stent 1 im Bereich der Linie V-V aus Figur 1, bei dem jeder rohrförmige Abschnitt 8 acht Elemente 10 aufweist. Von diesen acht Elementen 10 ist jedes zweite über Verbindungsstege 20 mit Elementen eines benachbarten Abschnittes 8 verbunden. Insgesamt sind also, wie dies durch die größeren Punkte 20 angedeutet ist, vier Elemente 10 über Verbindungsstege 20 mit Elementen des benachbarten Abschnittes verbunden, sodass sich eine vierzählige Symmetrieachse 38 der rohrförmigen Abschnitte 8 ergibt.

Figur 6 zeigt eine ebenfalls stark schematisierte Projektion zweier benachbarter rohrförmiger Abschnitte 8 eines weiteren erfindungsgemäßen Stents. Die Abschnitte 8 entsprechen dabei den Abschnitten 8 gemäß Figur 5. Sie sind jedoch zueinander um 45° bzw. eine halbe Periode der umlaufende Randkonturen der Abschnitte 8 verdreht. Auch hier symbolisieren die größeren Punkte 20 die mit Verbindungsstegen 20 verbundenen Elemente 10.

Es handelt sich bei den Figuren 5 und 6, wie erwähnt, um stark schematisierte Darstellungen. Die dargestellten Konturen sind lediglich Platzhalter für die geschnittenen Teile des betreffenden Stents und geben nicht deren tatsächliche Schnittkontur wieder.

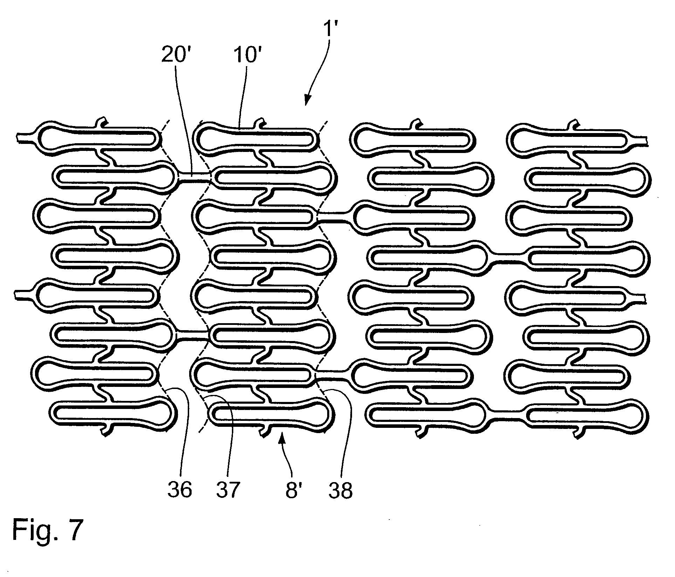

Figur 7 zeigt eine weitere bevorzugte Ausführungsform des erfindungsgemäßeri Stents 1' mit acht Elementen 10' pro rohrförmigen Abschnitt 8'. Diese Ausführungsform gleicht in ihrem grundsätzlichen Aufbau derjenigen aus Figur 1, sodass die lediglich auf die Unterschiede eingegangen werden soll.

Der Unterschied besteht darin, dass zwischen zwei benachbarten rohrförmigen Abschnitten 8' des Stents 1' jeweils nur zwei Verbindungsstege 20' vorgesehen sind, wobei an jedem vierten Element 10' ein Verbindungssteg zu dem in einer ersten Richtung angrenzenden rohrförmigen Abschnitt 8' angreift. Die Verbindungsstege 20' sind weiterhin von Abschnitt 8' zu Abschnitt 8' um eine halbe Periode der durch die gestrichelten Konturen 36 bis 38 angedeuteten, wellenartigen Randkontur der Abschnitte 8' versetzt. Durch diese versetzte Anordnung der Verbindungsstege 20' ergibt sich ein über seine Länge in alle Raumrichtungen flexibler Stent.

Wie schon bei der Ausführung aus Figur 1 sind auch bei dieser Variante die Elemente 10' eines jeden rohrförmigen Abschnittes 8' so Längsrichtung des Stents 1' zueinander versetzt angeordnet, dass ihre Enden die beschriebenen, miteinander in Phase bezüglich der Umfangsrichtung des Stents wellenartig umlaufenden Randkonturen 37 und 38 definieren. Weiterhin sind auch hier die rohrförmigen Abschnitte 8' so zueinander angeordnet, dass aneinander angrenzende Randkonturen 36 und 37 benachbarter Abschnitte 8' miteinander in Phase verlaufen, wodurch erfindungsgemäß die besonders gute und gleichmäßige Abdeckung der Gefäßinnenwand im expandierten Zustand erzielt wird.

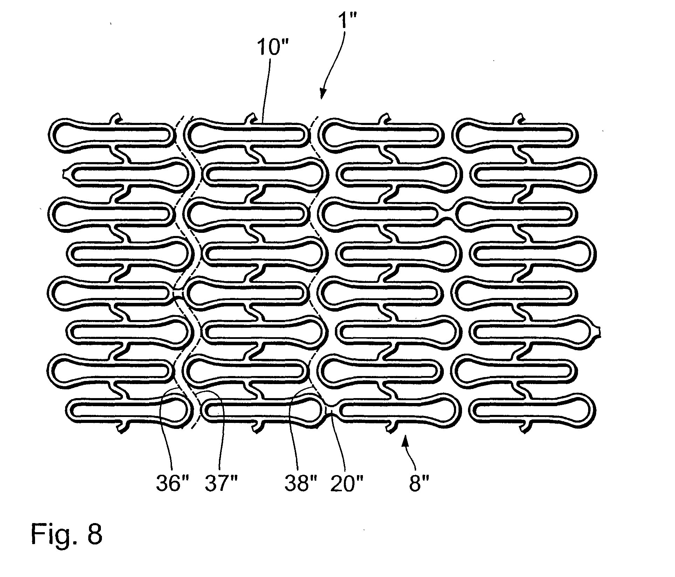

Figur 8 zeigt eine weitere bevorzugte Ausführung des erfindungsgemäßen Stents 1" mit acht Elementen 10" pro rohrförmigen Abschnitt 8". Diese Ausführung gleicht in ihrem grundsätzlichen Aufbau derjenigen aus Figur 7, sodass die lediglich auf die Unterschiede eingegangen werden soll.

Ein Unterschied besteht darin, dass zwischen zwei benachbarten rohrförmigen Abschnitten 8" des Stents 1'' jeweils nur ein Verbindungssteg 20" vorgesehen ist. Die Verbindungsstege 20" sind weiterhin von Abschnitt 8' zu Abschnitt 8' um eineinhalb Perioden der durch die gestrichelte Konturen 36" bis 38" angedeuteten, wellenartigen Randkontur der Abschnitte 8" versetzt. Durch die geringe Anzahl sowie die versetzte Anordnung der Verbindungsstege 20" ergibt sich ein über seine Länge in alle Raumrichtungen äußerst flexibler Stent.

Ein weiterer Unterschied besteht darin, dass der Abstand in Längsrichtung zwischen den rohrförmigen Abschnitten 8" so gering gewählt ist, dass die Randkonturen 36" und 37" benachbarter rohrförmiger Abschnitte 8" nach Art einer Verzahnung ineinander greifen. Hierbei kommen die einzelnen Elemente 10" des einen rohrförmigen Abschnittes 8" mit anderen Worten so nahe an die Elemente 10" des benachbarten rohrförmigen Abschnittes 8" heran, dass sich die benachbarten Elemente 10'' in Längsrichtung des Stents 1" teilweise überlappen. Bei dieser Gestaltung ergeben sich besonders kleine, gleichmäßige Lücken in der expandierten Stentstruktur und damit eine äußerst gute Abdeckung der Gefäßinnenwand.

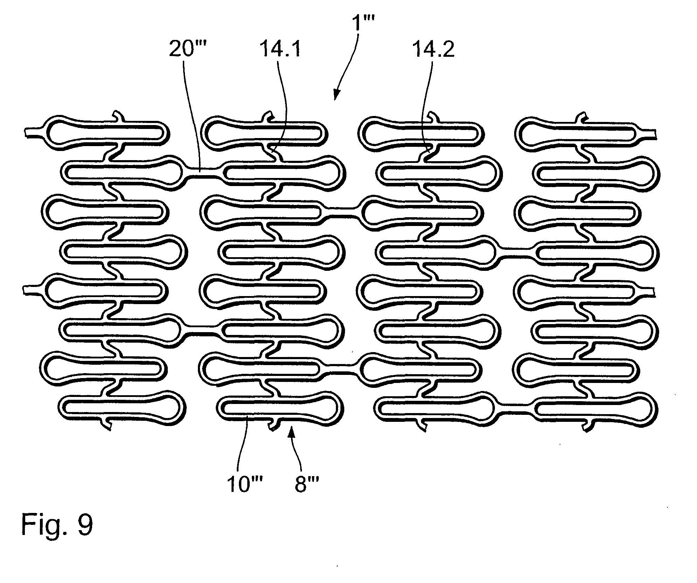

Figur 9 zeigt eine andere bevorzugte Variante des erfindungsgemäßen Stents 1"', welche sich von der Variante aus Figur 7 lediglich dadurch unterscheidet, dass in dieselbe Umfangsrichtung weisende zweite Verbindungsstege 14.1 und 14.2 in Längsrichtung des Stents 1''' aneinandergrenzender Elemente 10''' jeweils in entgegengesetzter Weise zur Umfangsrichtung geneigt angeordnet sind. Zusammen mit der bereits zu Figur 1 beschriebenen, um eine halbe Periode der Randkonturen versetzte Anordnung der ersten Verbindungsstege 20"' wird bei dieser Gestaltung eine gute Kompensation der Verkürzung des Stents der Expandieren erzielt.

Dies resultiert aus der sich beim Expandieren ergebende Längsverschiebung der einzelnen Elemente 10''', die wiederum aus der Ausrichtung der zweiten Verbindungsstege 14''' in Umfangsrichtung des Stents 1''' resultiert. Diese Längsverschiebung wird durch die dargestellte Gestaltung über den gesamten Stent aufaddiert, wodurch die durch die Verkürzung der einzelnen Elemente 10''' bei der Expansion des Stents bedingte Verkürzung des Stents auf ein Minimum, gegebenenfalls sogar auf Null reduziert werden kann.

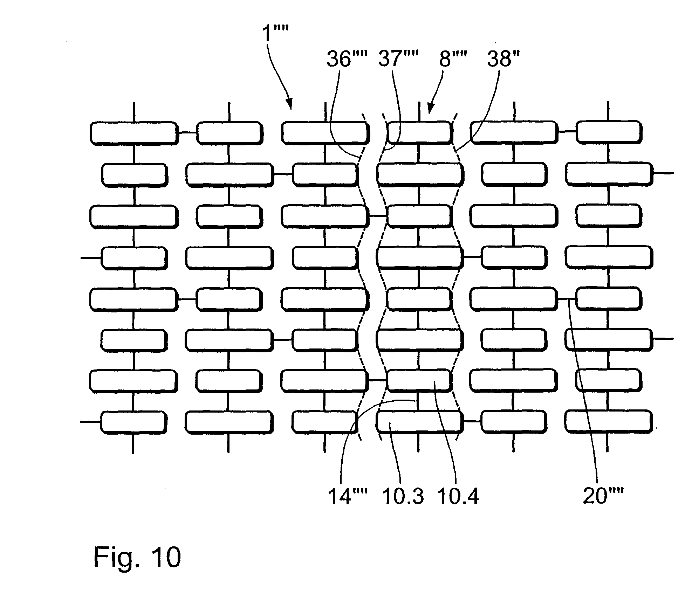

Figur 10 zeigt eine stark schematisierte Abwicklung der Mantelfläche einer weiteren bevorzugten Ausführung des erfindungsgemäßen Stents 1'''' mit die rohrförmigen Abschnitte 8"" bildenden Elementen 10'''', wobei zwei unterschiedliche Elementtypen 10.3 und 10.4 vorgesehen sind. Hierbei sind die Elemente 10.3 und 10.4 nicht in ihrer tatsächlichen Gestalt dargestellt sondern lediglich durch Zellen unterschiedlicher Größe repräsentiert. Die Elemente 10.3 und 10.4 können sich dabei sowohl in ihrer Gestalt als auch lediglich in ihren Abmessungen unterscheiden.

Der wesentliche Unterschied dieser Variante zu den vorbeschriebenen Ausführungsbeispielen der Erfindung liegt in der Tatsache, dass die rohrförmigen Abschnitte 8"" bei dieser Gestaltung auf Grund der unterschiedlichen Abmessungen der Elemente 10.3 und 10.4 in Längsrichtung des Stents 1"" wellenartig umlaufende Randkonturen 37"" und 38"" aufweisen, die zueinander um eine halbe Periode versetzt umlaufen. Die bezüglich der Längsrichtung des Stents 1"" gegebenen Mittelpunkte der Elemente 10.3 und 10.4 sind in Umfangsrichtung des Stents miteinander fluchtend angeordnet, sodass die Randkonturen 37"" und 38"" mit derselben Amplitude umlaufen.

Ansonsten ist auch hier wieder die erfindungsgemäße Gestaltung realisiert, bei der die benachbarten Randkonturen 36"" und 37"" zweier rohrförmiger Abschnitte 8"" zueinander in Phase umlaufen. Ebenso ist auch hier wieder die um eine halbe Periode der Randkonturen versetzte Anordnung der ersten Verbindungsstege 20"" realisiert. Die zweiten Verbindungsstege 14"" sind in Figur 10 lediglich schematisch dargestellt. Es versteht sich, dass diese in derselben Weise wie zu den obigen Beispielen beschrieben angeordnet bzw. gestaltet sein können. Insbesondere kann auch hier wieder analog zu der bereits oben beschriebenen Gestaltung eine entsprechende Verkürzungskompensation erzielt werden.

Zusammenfassend lässt sich feststellen, dass auch mit dieser Gestaltung dieselben Vorteile wie zu den oben beschriebenen Gestaltungsvarianten erzielt werden können.

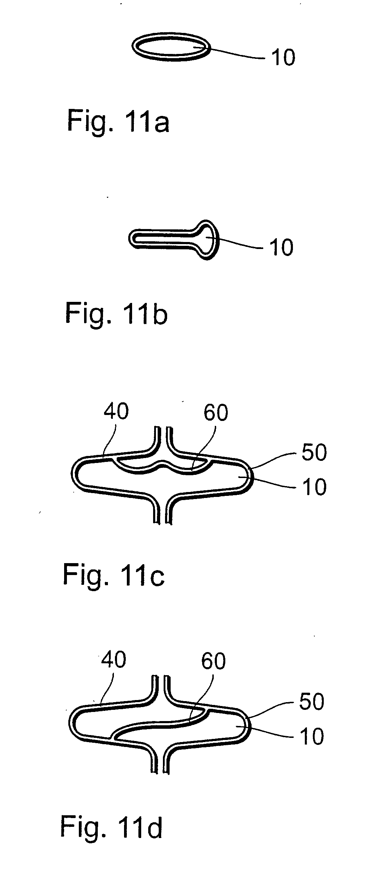

Die Figuren 11a bis 11d zeigen verschiedene Formen von Elementen 10, wie sie in den erfindungsgemäßen Stents der obigen Figuren 1 und 7 bis 10 - insbesondere im Stent der Figur 10 - an Stelle der dort dargestellten Elemente 10 bis 10'''' in den rohrförmigen Abschnitten benachbart zueinander zum Einsatz kommen können.

Die Ausführungsformen gemäß den Figuren 7a und 7b weisen eine vollständig geschlossene Struktur auf, während die Ausführungsformen der Figuren 7c und 7d zwei äußere Stege 40 und 50 aufweisen, die für sich genommen keine geschlossene Struktur ergeben, jedoch durch einen Verbindungssteg 60 ebenfalls geschlossene Elementflächen bzw. eine geschlossene zellenförmige Einheit bilden.

Die erfindungsgemäßen Stents wurden vorstehend anhand von Beispielen mit annähernd sinusförmig umlaufenden Randkonturen beschrieben. Es versteht sich jedoch, dass bei anderen Varianten der Erfindung auch andere Gestaltungen mit weniger gleichmäßig wellenartig umlaufenden Randkonturen ihre Anwendung finden können. Insbesondere muss die Anordnung der die rohrförmigen Abschnitte bildenden Elemente nicht notwendigerweise jeweils abwechselnd erfolgen. Die Elemente können beispielsweise auch in Gruppen angeordnet sein, die im Sinne der Beispiele aus den Figuren 1 und 7 bis 9 wechselweise versetzt zueinander angeordnet sind und die zusätzlich oder alternativ im Sinne des Beispiels aus der Figur 10 unterschiedliche Abmessungen der Längsrichtung des Stents aufweisen. Hierbei können die Gruppen auch eine unterschiedliche Anzahl von Elementen und zusätzlich oder alternativ auch unterschiedliche Arten von Elementen enthalten.

## Patentansprüche

1. Stent, insbesondere Koronarstent, mit wenigstens zwei in Längsrichtung des Stents (1; 1'; 1"; 1'''; 1'''') benachbart angeordneten, rohrförmigen Abschnitten (8; 8'; 8"; 8"'; 8"") aus mehreren miteinander verbundenen, im wesentlichen zellenförmigen und eine Orientierung aufweisenden Elementen (10; 10'; 10"; 10'''; 10''''), die in Längsrichtung des Stents (1 ; 1'; 1''; 1'''; 1"") über mindestens ein erstes Verbindungsmittel (20; 20'; 20"; 20'''; 20"") miteinander verbunden sind, wobei die Elemente (10; 10'; 10''; 10'''; 10'''') derart angeordnet und/oder ausgebildet sind, dass die Längsrichtung des Stents (1; 1'; 1"; 1'''; 1'''') liegenden Enden der Elemente (10; 10'; 10"; 10'''; 10'''') eine in Umfangsrichtung des Stents (1; 1'; 1''; 1'''; 1'''') wellenartig umlaufende Randkontur (36, 37, 38; 36", 37", 38"; 36"", 37"", 38"") definieren, **dadurch gekennzeichnet, dass** die aneinander angrenzenden Randkonturen (36, 37; 36", 37"; 36"", 37"") zweier rohrförmiger Abschnitte (8; 8'; 8"; 8"'; 8"") im wesentlichen phasengleich umlaufen.

2. Stent nach Anspruch 1, **dadurch gekennzeichnet, dass** die Randkonturen (36, 37; 36", 37"; 36'''', 37'''') der beiden rohrförmigen Abschnitte (8; 8'; 8"; 8"'; 8"") nach Art einer Verzahnung ineinander greifen.

3. Stent nach Anspruch 1 oder 2, **dadurch gekennzeichnet, dass** die beiden Randkonturen (37, 38; 37", 38"; 37"", 38"") eines rohrförmigen Abschnitts (8; 8'; 8"; 8"'; 8"") zueinander im wesentlichen in Phase oder zueinander im wesentlichen um eine halbe Periode versetzt verlaufen.

4. Stent nach einem der vorhergehenden Ansprüche ,**dadurch gekennzeichnet, dass** das erste Verbindungsmittel (20; 20'; 20"; 20'''; 20"") Elemente (10; 10'; 10"; 10'''; 10'''') gleicher Orientierung miteinander verbindet,.

5. Stent nach einem der vorhergehenden Ansprüche, **dadurch gekennzeichnet, dass** das erste Verbindungsmittel (20; 20'; 20"; 20'''; 20"") stegartig ausgebildet ist.

6. Stent nach einem der vorhergehenden Ansprüche, **dadurch gekennzeichnet, dass** das erste Verbindungsmittel (20; 20'; 20"; 20'''; 20"") im wesentlichen parallel zur Längsachse des Stents (1; 1'; 1"; 1'''; 1'''') verläuft.

7. Stent nach einem der vorhergehenden Ansprüche, **dadurch gekennzeichnet, dass** höchstens zwei, bevorzugt ein erstes Verbindungsmittel (20; 20'; 20"; 20'''; 20"") zum Verbinden benachbarter rohrförmiger Abschnitte (8; 8'; 8"; 8"'; 8"") vorgesehen ist.

8. Stent nach einem der vorhergehenden Ansprüche, **dadurch gekennzeichnet, dass** mehr als zwei rohrförmige Abschnitte (8; 8'; 8"; 8"'; 8"") vorgesehen sind und die ersten Verbindungsmittel (20'; 20"; 20'''; 20"") über die Länge des Stents (1'; 1''; 1'''; 1'''') von Abschnitt (8'; 8"; 8'''; 8"") zu Abschnitt (8'; 8"; 8'''; 8"") in Umfangsrichtung des Stents (1'; 1"; 1'''; 1'''') versetzt, bevorzugt um wenigstens eine halbe Periode der Randkontur (36, 37, 38; 36", 37", 38"; 36'''', 37'''', 38"") versetzt angeordnet sind.

9. Stent nach einem der vorhergehenden Ansprüche, **dadurch gekennzeichnet, dass** die Elemente (10''') wenigstens eines Abschnittes (8"") in Umfangsrichtung des Stents (1''') über zur Umfangsrichtung geneigt angeordnete, bevorzugt S-förmig verlaufende, zweite Verbindungsmittel (14.1, 14.2) verbunden sind, wobei in dieselbe Umfangsrichtung weisende zweite Verbindungsmittel (14.1,14.2) in Längsrichtung des Stents aneinandergrenzender Elemente (10''') in entgegengesetzter Weise zur Umfangsrichtung geneigt angeordnet sind.

10. Dilatationskatheter mit einem Stent (1; 1'; 1"; 1'''; 1'''') nach einem der vorstehenden Ansprüche.
